# EUROPEAN PATENT SPECIFICATION

(11) **EP 4 547 213 B1**
(45) Date of publication and mention of the grant of the patent: **14.01.2026**
(21) Application number: 23735275.2
(22) Date of filing: 23.06.2023
(51) Int. Cl.: A61K 9/08, A61K 47/08, A61K 47/10, A61K 47/26, A61K 8/368, A61K 8/60, A61K 8/86, A61Q 19/00, A61K 8/34

(54) **ANTI-ACNE COMPOSITION**
ANTI-AKNE-ZUSAMMENSETZUNG
COMPOSITION ANTI-ACNÉ

(30) Priority: 29.06.2022 EP 22181944
(43) Date of publication of application: 07.05.2025
(73) Proprietor: Unilever IP Holdings B.V., 6708 WH Wageningen (NL); Unilever Global IP Limited, Wirral, Merseyside CH62 4ZD (GB)
(72) Inventor: MORIKIS, Thomas, Nikolaos, 6708 WH Wageningen (NL); WOLCHESKI, Jeffrey, Scott, 6708 WH Wageningen (NL)
(74) Representative: James, Helen Sarah
(86) International application number: PCT/EP2023/067109
(87) International publication number: WO 2024/002892

(56) References cited:
- EP-A2- 1 172 087
- WO-A2-2010/087964

## Description

### Field of the invention

The present invention is directed to a topical composition that provides anti-acne benefits, without the need for a traditional topical ointment. More particularly, the invention is directed to a composition that comprises a) 90%-93% by weight water; b) 1% by weight to 3% by weight of a non-ionic surfactant having an HLB from 15.7 to 17.5; and c) from 0.75% to 2% by weight of an anti-acne agent, wherein the anti-acne agent is salicylic acid wherein the composition is substantially free of thickeners and gelling agents, preferably having 0 wt% of thickeners and gelling agents; and wherein the composition is a stable micellar water which is a continuous monophasic water phase.

### Background of the Invention

The present invention relates to a topical composition and a method of reducing, treating, controlling and/or preventing occurrence of acne on skin.

Acne, also known as acne vulgaris, is a common skin condition that affects nearly all adolescents and adults at some times in their lives. It has a complex etiology, involving abnormal keratinization, excess sebum production, androgen function, bacterial growth, and immune hypersensitivity. Other factors which have been linked to acne are presence of free radicals with subsequent oxidative stress leading to cellular damage.

The earliest acne lesion is known as the microcomedone. These evolve into comedones which may either be open ("blackhead") or closed ("whitehead"). The various stages of acne have been classified as comedones, papules, pustules, and cysts.

It has been observed that acne usually occurs in areas rich in sebaceous glands like the face, neck and back. A bacteria *Propionibacterium acne (P. acnes)* has also been implicated in occurrence of acne.

Many products are marketed for the treatment of acne, including the ingestion of pills and topical applications of ointments (e.g., gels and creams). Typically, anti-acne agents, such as salicylic acid are known for use in topical application; yet it is also known that salicylic acid is difficult to formulate, especially in water-based products. Topical applications are popular among consumers, yet continuous and repeated use of such products can lead to dry and irritated skin, typically as a result of the anti-acne agent. Accordingly, there remains a need to topically deliver the benefits of anti-acne agents, such as salicylic acid, without the unfavorable side effects.

Micellar water is a product which is known to be a gentle and mild cleanser. It is popular among consumers as it can quickly and easily clean skin, without the need to rinse. Moreover, micellar water well-suited for many skin types, including dry and oily skin. Thus, micellar water is typically used to remove substances from the skin, including dirt, oil and makeup, and can help promote skin clarity, but for many consumers, acne remains a problem and there is a need to also apply an additional topical treatment for acne. Patent document EP1172087 discloses topical formulations comprising salicylic acid.

The inventors of the present application have surprisingly found that it was possible to include an anti-acne agent in a micellar water, so that consumers may treat acne, while lessening the unfavorable side effects from conventional topicals ointments (e.g., gels and creams). In other words, it was surprisingly found that it was possible to achieve transparent and stable micellar water with an anti-acne agent, such as salicylic acid, which provides a more gentle and milder format for reducing, treating, controlling and/or preventing the occurrence of acne.

### Summary of the Invention

In a first aspect, a composition comprises a) 90%-93% by weight water; b) 1% by weight to 3% by weight of a non-ionic surfactant having an HLB from 15.7 to 17.5; and c) from 0.75% to 2% by weight of an anti-acne agent, wherein the anti-acne agent is salicylic acid wherein the composition is substantially free of thickeners and gelling agents, preferably having 0 wt% of thickeners and gelling agents; and wherein the composition is a stable micellar water which is a continuous monophasic water phase.

In a second aspect, is a non-therapeutic method for reducing, treating or preventing the occurrence of acne comprising: a) applying the composition as defined above onto a surface of skin; b) optionally, rubbing the composition into the surface of the skin; and c) optionally repeating step (a), and optionally step (b), daily, twice a day, or three times a day.

In another aspect, is a non-therapeutic use of the composition as defined above as an anti-acne composition or to obtain an anti-acne benefit.

All other aspects of the present invention will more readily become apparent from the description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of any of the compositions or components thereof, unless otherwise specified. It should be noted that in specifying any ranges of values, any particular upper value can be associated with any particular lower value.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The disclosure, as found herein, is to be considered to cover all embodiments as found in the claims as being multiply dependent upon each other irrespective of the fact that claims may be found without multiple dependency or redundancy.

Skin, as used herein, includes skin on the face, neck, chest, back, arms (including underarms), axilla, buttocks, hands, legs and scalp.

Acne, as used herein, refers to, for example, acne lesions such as microcomedones, open comedones ("blackhead"), closed comedones ("whitehead"), papules, pustules, and cysts. Treating, preventing, controlling and/or reducing acne may refer to the appearance and/or lessening the appearance of acne lesions on a skin surface (e.g., size, type of lesion, and/or color) and/or the number of acne lesions on skin. As used herein, an "anti-acne benefit" may refer to killing the bacteria known to cause acne, reducing the number of acne lesions, reducing the appearance of acne lesions on skin, removing excess oil from the skin, promote growth of new skin cells and/or the removal of dead skin cells.

Skin benefit agent, as used herein, refers to an improvement in a facial or body characteristic after topical application, for example, even skin tone, reduction in wrinkles, or the like.

Derivative(s), as used herein, refers to functional group substitutions on a compound.

Micelle, as used herein, is an aggregate assembly of surfactant molecules dispersed in a liquid (e.g., water). Micellar water, as used herein, is a stable dispersion or emulsion that is typically transparent and/or translucent which contain micelles. As used herein, stable refers to a micellar water which is a continuous (monophasic) water phase rather than, e.g., a bi-phasic composition.

In a first aspect, a composition comprises a) 90%-93% by weight water; b) 1% by weight to 3% by weight of a non-ionic surfactant having an HLB from 15.7 to 17.5; and c) from 0.75% to 2% by weight of an anti-acne agent, wherein the anti-acne agent is salicylic acid wherein the composition is substantially free of thickeners and gelling agents, preferably having 0 wt% of thickeners and gelling agents; and wherein the composition is a stable micellar water which is a continuous monophasic water phase. aspect, the non-ionic surfactant having an HLB of at least 15.7 is ceteth-20, laureth-23, polysorbate-20, and/or mixtures thereof, preferably polysorbate-20.

In one aspect, the composition, as described herein, has at least one anti-acne agent. An anti-acne agent, as described herein, includes but is not limited to: salicylic acid, benzoyl peroxide, sulfur, thymol, willow bark extract, succinic acid, azelaic acid and/or mixtures thereof. In one aspect, the anti-agent agent is salicylic acid, thymol, willow bark extract and/or mixtures thereof. According to the claims, the anti-agent agent is salicylic acid in an amount from 0.75% to 2% by weight.

In one aspect, a weight ratio of the non-ionic surfactant having an HLB of at least 15.7 to the anti-acne agent, is about 5:1 to about 1:1, or about 4:1 to about 1:1, or about 3:1 to about 1:1, or about 2:1 to about 1:2, or about 2:1 to about 1:1, or about 5:1, or about 3:1, or about 2.5:1, or about 2:1, or about 1.5:1, or about 1:1, including all ratios subsumed therein.

In one aspect, the pH of the composition, as described herein, is from 3.0 to 7.0, or from 3.5 to 6.5, or from 3.8 to 6.2, or from 4.0 to 6.0, or from 4.0 to 5.5, or from 4.25 to 5.75, or from 4.25 to 5.25. Adjusters suitable to modify the pH of the composition as described herein may be used. Such pH adjusters include but are not limited to triethylamine, NaOH, KOH, H₂SO₄, HCl, C₆H₈O₇ (i.e., citric acid) and/or mixtures thereof. Buffers, like potassium oxide, to stabilize pH are also suitable for use. The pH of the composition is assessed by using conventional instrumentation such as a pH meter made commercially available from Thermo Scientific^{®}.

In one aspect, the composition, as described herein but not claimed, comprises at least 88% water,
preferably 88%-94% by weight water; (b) at least 0.5% by weight a non-ionic surfactant having an HLB of at least 15.7, wherein the non-ionic surfactant is ceteth-20, laureth-23, polysorbate-20, and/or mixtures thereof; and (c) an anti-acne agent, wherein the anti-acne agent is salicylic acid. Surprisingly and unexpectedly, the compositions described herein may be in the form of a stable micellar water (e.g., not bi-phasic) with none of the anti-acne agent precipitating from the continuous water phase of the micellar water.

### Optional Surfactants

It is within the scope of this invention to include optional surfactants along with the non-ionic surfactants having an HLB of at least 15.7, as described herein. The optional surfactants may be other non-ionic surfactants, for example, having an HLB of 15.6 or less.

Other optional surfactants can include those which are amphoteric (which depending on pH can be zwitterionic). Illustrative optional amphoteric surfactants suitable for use generally include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants that may be used include sodium lauroamphoacetate, sodium cocoamphoacetate, sodium lauroamphoacetate, sodium cocoamphoacetate and/or mixtures thereof or the like.

As to zwitterionic surfactants that may optionally be used in the composition described herein, such surfactants typically include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms and generally comply with an overall structural formula:
R¹--[--C(O)--NH(CH₂)_{q}--]ᵣ--W(R²)(R³)A--B where R¹ is alkyl or alkenyl of 7 to 18 carbon atoms; R² and R³ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants for use in the composition, as described herein, and within the above general formula include simple betaines of formula:

R¹--N⁺(R²)(R³)-CH₂CO₂⁻

and amido betaines of formula:
R¹--CONH(CH₂)ₜ--N⁺--(R²)(R³)CH₂CO₂⁻ where t is 2 or 3.

In both formulae R¹, R² and R³ are as defined previously. R¹ may, in particular, be a mixture of C₁₂ and C₁₄ alkyl groups derived from coconut oil so that at least half, preferably at least three quarters of the groups R¹ have 10 to 14 carbon atoms. R² and R³ are preferably methyl.

A further possibility is that the zwitterionic surfactant is a sulphobetaine of formula:

R¹ --N⁺(R²)(R³)-(CH₂)₃SO₃⁻

or

R¹--CONH(CH₂)ᵤ --N⁺(R²)(R³)-(CH₂)₃SO₃⁻

where u is 2 or 3, or variants of these in which --(CH₂)₃SO₃⁻ is replaced by-CH₂C(OH)(H)CH₂SO₃⁻.

In these formulae, R¹, R² and R³ are as previously defined.

Illustrative examples of the zwitterionic surfactants include betaines like cocodimethyl carboxymethyl betaine, cocoamidopropyl betaine and laurylamidopropyl betaine. An additional zwitterionic surfactant suitable for use includes cocoamidopropyl sultaine. Such surfactants are made commercially available from suppliers like Stepan Company, and it is within the scope of the invention to optionally employ mixtures of the same.

Optional surfactants may also include cationic surfactants. One class of cationic surfactants includes heterocyclic ammonium salts such as cetyl or stearyl pyridinium chloride, alkyl amidoethyl pyrrylinodium methyl sulfate, and lapyrium chloride. Tetra alkyl ammonium salts are another useful class of cationic surfactants for use. Examples include cetyl or stearyl trimethyl ammonium chloride or bromide, such as cetyltrimethylammonium chloride; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride. Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate. Still other useful cationic surfactants include quaternized hydrolysates of silk, wheat, and keratin proteins, and it is within the scope of the cleansing composition to use mixtures of the aforementioned cationic surfactants. In one aspect, when used, the cationic surfactant is cetrimonium chloride, cetylpyridinium chloride and/or mixtures thereof.

Optional surfactants may also include anionic surfactants. Illustrative examples of anionic surfactants include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic surfactant may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium.

The anionic surfactant may also be alkyl sulfosuccinates (including mono- and dialkyl, e.g., C₆-C₂₂ sulfosuccinates); alkyl and acyl taurates (often methyl taurates), alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, and the like.

Sulfosuccinates may be monoalkyl sulfosuccinates having the formula:

R¹OC(O)CH₂CH(SO₃M)CO₂M;

and amide-MEA sulfosuccinates of the formula:

R¹CONHCH₂CH₂OC(O)CH₂CH(SO₃M)CO₂M

wherein R¹ ranges from C₈-C₂₂ alkyl.

Sarcosinates are generally indicated by the formula:
R²CON(CH₃)CH₂CO₂M, wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described.

The composition disclosed herein may contain C₈-C₁₈ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described U.S. Pat. No. 5,393,466, which is hereby incorporated by reference. This compound has the general formula:

R⁵C-(O)O-C(X)H-C(Y)H-(OCH₂-CH₂)M-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an aspect of the composition, the anionic surfactant, if used, is 2-acrylamido-2-methylpropane sulfonic acid, ammonium lauryl sulfate, ammonium perfluorononanoate, potassium lauryl sulfate, sodium alkyl sulfate, sodium dodecyl sulfate, sodium laurate, sodium laureth sulfate, sodium lauroyl sarcosinate, sodium stearate, sodium sulfosuccinate esters, sodium lauroyl isethionate, or a combination thereof. Such anionic surfactants are commercially available from suppliers like Galaxy Surfactants, Clariant, Sino Lion, Stepan Company, and Innospec.

When present, the amount of optional surfactants included, by weight of the composition described herein, is collectively no more than 4%, or no more than 3%, or no more than 2%, or no more than 1%. In one aspect, the amount of optional surfactants included, by weight of the composition described herein, is collectively about 0.001% to about 4%, or about 0.01% to about 4%, or about 0.1% to about 2%, or about 0.1% to about 1%, or about 0.1% to about 0.75%, including all ranges and amounts subsumed therein.

### Emollients

In one aspect, emollients can be included in the composition disclosed herein. These include ester of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and/or mixtures thereof. In one aspect, the emollient is caprylic/capric triglyceride, PEG-6 caprylic/capric glyceride and/or mixtures thereof. In one aspect, the amount of emollient, if included, by weight of the composition described herein, is collectively about 0.001% to about 4%, or about 0.01% to about 4%, or about 0.1% to about 2%, or about 0.1% to about 1%, or about 0.1% to about 0.75%, including all ranges and amounts subsumed therein.

### Humectants

In one aspect, humectants, such as water-soluble polyols, can be included in the composition disclosed herein. These include sorbitol, glycerol (or glycerine), mannitol, xylitol, maltitol and/or mixtures thereof. Other humectants include propylene glycol, dipropylene glycol, polypropylene glycol (e.g., PPG-9), polyethylene glycol, hydroxypropyl sorbitol, hexylene glycol, 1,3-butylene glycol, propanediol, 1,2-octane diol, 1,2-hexane diol, isoprene glycol, 1,2,6-hexanetriol, ethoxylated glycerol, propoxylated glycerol and/or mixtures thereof. In one aspect, the humectant is glycerol. In one aspect, the humectant is a combination of glycerol and hexylene glycol.

In one aspect, the amount of humectant(s) included, by weight of the composition described herein, is collectively about 0.1% to about 4.5%, or about 0.5% to about 4.5%, or about 1% to about 4.5%, or about 1% to about 4%, or about 1.5% to about 3.5%, or about 2.5% to about 3.5%, including all ranges and amounts subsumed therein.

In one aspect, the humectant is a combination of hexylene glycol and glycerol, at a weight ratio of about 1:5 to about 5:1, or about 1:4 to about 4:1, or about 1:3 to about 3:1, including all ratios subsumed therein. In one aspect, a weight ratio of hexylene glycol to glycerol is about 5:1, or about 3:1, or about 2.5:1

### Preservatives

In one aspect, preservatives can be included in the composition disclosed herein. As to illustrative preservatives suitable for use, these include sodium benzoate, iodopropynyl butyl carbamate, phenoxyethanol, hydroxyacetophenone, ethylhexylglycerine, methyl paraben, propyl paraben, imidazolidinyl urea, sodium dehydroacetate, dimethyl-dimethyl (DMDM) hydantoin,benzyl alcohol and/or mixtures thereof. Other preservatives suitable for use include sodium dehydroacetate, chlorophenesin and decylene glycol. The amount of preservatives(s) included, by weight of the composition described herein, is collectively about 0.01% to 2.0%, or about 0.1% to about 1%, or about 0.1% to about 0.75%, including all ranges subsumed therein.

### Optional Additives

Fragrances, fixatives, opacifiers (like titanium dioxide), chelators (like EDTA) may optionally be included in the composition as disclosed herein. The amount of optional fragrances, fixatives, opacifiers, and chelators included, by weight of the composition described herein, is collectively from about 0.03% to 3%, or from about 0.1 and 2.6%, including all ranges subsumed therein.

Another optional additive suitable for use includes hemp oil with 2.5 to 25% by weight cannabigerol and/or cannabidiol at from 0.5% to 10% by weight. When optionally used, such oil makes up from 0.0001 to 1.5% by weight of the composition, and preferably, from 0.01 to 1% by weight of the composition, including all ranges and amounts subsumed therein.

Film forming agents may be used in the compositions of the present invention. While optional, such agents can aid with composition adhering to the surface they are applied to. Film forming agents include those having hydrophilic properties and they include materials comprising polyvinylpyrrolidone (PVP), acrylates, acrylamides, and copolymers thereof. Deposition agents like organosiloxanes may also be used. When optionally used, such agents make up from 0.001% to 1% by weight of the composition, including all ranges and amounts subsumed therein.

In one aspect, the composition as disclosed herein is substantially free of thickeners or gelling agents, such as silica, sodium carboxymethyl cellulose and/or a carbomer. In another aspect, the composition as disclosed herein is substantially free of parabens, silicones and/or sulfates. As used herein, substantially free refers less than 0.5% by weight of the composition, or less than 0.1% by weight of the composition, preferably 0% by weight of the composition.

### Optional Skin Benefit Agents

Optional skin benefit agents that may be provided in the composition herein include: water-soluble moisturizing agent, occlusive agents, plant extracts, vitamins and minerals, resorcinols, optical agents, even tone agents, anti-inflammatory agents, photostabilizers, antioxidants, wrinkle-reducing agents, desquamation promoters, and/or mixtures thereof or the like. Each of these substances may be present in an amount of about 0.05% to about 5%, or about 0.1% to about 4%, or about 0.1 to about 2% by weight of the composition, including all ranges and amounts subsumed therein.

Water soluble moisturizing agents are suitable for optional skin benefit agents. These include dicaprylyl carbonate as well as substituted ureas like hydroxymethyl urea, hydroxyethyl urea, hydroxypropyl urea; bis(hydroxymethyl)urea; bis(hydroxyethyl)urea; bis(hydroxypropyl)urea; N,N'-dihydroxymethyl urea; N,N'-di-hydroxyethyl urea; N,N'-dihydroxypropyl urea; N,N,N'-tri-hydroxyethyl urea; tetra(hydroxymethyl)urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea and/or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred for optional use is hydroxyethyl urea. The same is available as a 50% aqueous liquid from Nouryon under the trademark Hydrovance^{®}.

Vitamins and are suitable for optional skin benefit agents. Vitamins may be used like vitamin A (including retinoic acid precursors), vitamin B₂, picolinamide, niacinamide (vitamin B₃), panthenol (vitamin Bs), vitamin B₆, vitamin C (also including ascorbyl tetraisopalmitate, magnesium ascorbyl phosphate and ascorbyl glycoside), vitamin D, vitamin E, vitamin K, and/or mixtures or derivatives thereof or the like. The retinoic acid precursor may be retinol, retinal, retinyl propionate, retinyl palmitate, retinyl acetate and/or mixtures thereof. Still another retinoic acid precursor suitable for use is hydroxyanasatil retinoate made commercially available under the name Retextra^{®} as supplied by Molecular Design International.

Other optional skin benefit agents suitable for use include resorcinols like 4-hexyl resorcinol, 4-ethyl resorcinol, 4-phenylethyl resorcinol, 4-cyclopentyl resorcinol, 4-cyclohexyl resorcinol 4-isopropyl resorcinol, isobutylamido thiazolyl resorcinol (e.g., Thiamidol) and/or mixtures thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Patent Application Publication No. 2016/0000669A1.

Other suitable optional skin benefit agents include omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 12-hydroxystearic acid, conjugated linoleic acid, stearic acid, palmitic acid, lauric acid, hyaluronic acid and salts thereof (like Na+ and K+ salts of the same), ceramides (e.g. Ceramide 1, Ceramide 3, Ceramide 3B and Ceramide 6) and pseudoceramides, allantoin, pyroglutamic acid (PCA) salt derivatives including zinc PCA and sodium PCA, petroselinic acid, terpineol, limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil, and extracts like sage, aloe vera, green tea, sugar cane, citrus, grapeseed, thyme, chamomile, yarrow, cucumber, liquorice, rosemary extract and/or mixtures thereof or the like.

The composition, as described herein, may be in the form of a leave-on composition. As to packaging, the composition as described herein, can be packaged in a spray bottle, bottle with a self-foaming mechanism/pump, or provided as an impregnating wetting agent on cotton swabs, wipes, towelettes, cosmetic substrate sheets (like those described in U.S. Pat. No. 6,294,182) or the like. The spray bottle may also be metal and the composition may be provided via conventional aerosol packaging technologies and including those which utilize air-in-bag discharging cannisters, mechanisms and actuators. It is also within the scope of the invention to include foaming agents (e.g., zwitterionic and/or amphoteric surfactants) so that the composition can be discharged as a foam. In an embodiment of the invention, the composition is provided in a standard spray bottle with a spray actuator and sprayed on to a surface, especially skin.

A precursor, as used herein, means a composition which is a concentrate and suitable to be diluted (or hydrated) with water to produce a composition ready for use.

When making the precursor of the composition described herein, ingredients are mixed with moderate shear, under atmospheric conditions and at a temperature from 20 to 65°C. Depending on the desired volume, precursor (with less than 15% by weight water) should be mixed with water followed by moderate agitation to produce desired sanitizing composition. Precursor can be provided for in biodegradable packaging and the packaging used may be refillable or reusable, biodegradable, and/or at least 50%, or, at least 100% made from post-consumer recycled resin.

In another aspect, is a non-therapeutic method for reducing, treating or preventing the occurrence of acne comprising: a. applying the composition as defined in any of claims 1 to 13 onto a surface of skin; b. optionally, rubbing the composition into the surface of the skin; and c. optionally repeating step (a), and optionally step (b), daily, twice a day, or three times a day.

The non-therapeutic methods of reducing, treating, controlling and/or preventing the occurrence of acne does not require specialized equipment and can be determined by visually evaluating, for example, the difference in the number or size of the acne lesions, especially after a certain period of time, e.g., after at least one week, after at least two weeks, after at least three weeks, or after at least one month, or by evaluating the visual texture, color, and/or appearance of skin, especially after a certain period of time, e.g., after at least one week, after at least two weeks, after at least three weeks, or after at least one month.

In another aspect, is a non-therapeutic method for providing an anti-acne benefit to skin following applying a composition according to claims 1-13 to a desired skin surface, and optionally, rubbing the composition into the surface of the skin. Optionally, the method may be repeated daily, twice a day or three times a day.

The composition of the invention should be supplied with instructions to apply or spray, the composition on to a surface, for example, skin. Optionally, following the application of the composition, the user can be instructed to rub the composition into the surface. Further optionally, the user can be instructed to apply the composition daily, twice a day or three times a day.

The following Examples are provided to further illustrate an understanding of the invention. The Examples are not intended to limit the scope of the claims.

### Example 1

For each composition, water was heated to 80°C with mixing. At 80°C, EDTA, citric acid & salicylic acid were added and mixed for about 10 minutes or until all particles were dissolved. Then, sodium hydroxide was added and mixed, followed by slowly adding the non-ionic surfactant. Then, while cooling the solution to 36°C, hexylene glycol and glycerin were added, followed by an emollient, further followed by cationic surfactants, a preservative and niacinamide. Each composition was observed after being stored at room temperature overnight.

| **Ingredients** | | **Comp. A (wt%)** | **Comp. B (wt%)** | **Comp. C (wt%)** | **Comp. (wt%)** | **Comp. E (wt%)** |
|---|---|---|---|---|---|---|
| Water | | balance | balance | balance | balance | balance |
| Non-Ionic Surfactant | | | | | | |
| | Laureth-23 (HLB:16.9) | 1.5 | -- | -- | -- | -- |
| | Polysorbate 20 (HLB: 16.7) | -- | 1.5 | -- | - | -- |
| | Ceteth-20 (HLB: 15.7) | -- | -- | 1.5 | -- | -- |
| | Cetearyl Alcohol (HLB: 15.5) | -- | -- | -- | 1.5 | -- |
| | Cetearyl Alcohol (HLB 15.5) + Cetearyl Glucoside (HLB 11) Mixture | -- | -- | -- | -- | 1.5 |
| Emollient | | 0.5 | 0.5 | 0.5 | 0.5 | 0.5 |
| Anti-Acne Agent (salicylic acid) | | 1 | 1 | 1 | 1 | 1 |
| EDTA | | 0.13 | 0.13 | 0.13 | 0.13 | 0.13 |
| Hexylene Glycol | | 2.5 | 2.5 | 2.5 | 2.5 | 2.5 |
| Glycerin | | 1 | 1 | 1 | 1 | 1 |
| Catonic surfactants | | 0.15 | 0.15 | 0.15 | 0.15 | 0.15 |
| Niacinamide | | 0.01 | 0.01 | 0.01 | 0.01 | 0.01 |
| Preservative | | 0.2 | 0.2 | 0.2 | 0.2 | 0.2 |
| Citric Acid | | 0.15 | 0.15 | 0.15 | 0.15 | 0.15 |
| NaOH | | 0.6 | 0.6 | 0.6 | 0.6 | 0.6 |
| Result | | Clear transparent and stable - no precipitate | Clear transparent and stable-- no precipitate | Clear transparent and stable-- no precipitate | Unstable/ Bi-phasic | Unstable/ Bi-phasic |

As shown above, surprisingly and unexpectedly, only the compositions having 88%-94% by weight water, a non-ionic surfactant having an HLB of at least 15.7, and an anti-acne agent remained clear, transparent and stable with no precipitate. The compositions which lacked a non-ionic surfactant having an HLB of at least 15.7 were not stable and separated into two phases (e.g., bi-phasic).

## Claims

1. A composition comprising:
a) 90%-93% by weight water;
b) 1% by weight to 3% by weight of a non-ionic surfactant having an HLB from 15.7 to 17.5; and
c) from 0.75% to 2% by weight of an anti-acne agent, wherein the anti-acne agent is salicylic acid
wherein the composition is substantially free of thickeners and gelling agents, preferably having 0 wt% of thickeners and gelling agents; and
wherein the composition is a stable micellar water which is a continuous monophasic water phase.

2. The composition of claim 1, wherein the HLB of the non-ionic surfactant is from 15.7 to 16.9.

3. The composition according to claim 1 or 2, wherein the non-ionic surfactant is ceteth-20, laureth-23, polysorbate-20 and/or mixtures thereof, preferably polysorbate-20.

4. The composition according to any of the preceding claims, wherein the composition comprises 1.5% to 2.5% of the non-ionic surfactant.

5. The composition according to any of the preceding claims, wherein the composition comprises 0.001% to 2%, or 0.05% to 1.0%, or 0.5% to %, or 0.75% to 1.5%, or 0.75% to 1.25%, by weight of the anti-acne agent.

6. The composition according to any of the preceding claims, wherein the composition is substantially free of parabens, silicones and/or sulfates.

7. The composition according to any of the preceding claims, wherein a weight ratio of the non-ionic surfactant to the anti-acne agent is 5:1, or 3:1, or 1.5:1.

8. The composition according to any of the preceding claims, wherein the composition is transparent.

9. The composition according to any of the preceding claims further comprising at least one optional surfactant, preferably a cationic surfactant.

10. The composition according to any of the preceding claims further comprising a polyol and the polyol is sorbitol, glycerol, mannitol, xylitol, maltitol dipropylene glycol, polypropylene glycol, polyethylene glycol, hydroxypropyl sorbitol, hexylene glycol, 1,3-butylene glycol, 1,2-octane diol, 1,2-hexane diol, isoprene glycol, 1,2,6-hexanetriol, ethoxylated glycerol, propoxylated glycerol and/or mixtures thereof, preferably glycerol, hexylene glycol and/or mixtures thereof.

11. The composition according to any of the preceding claims further comprising at least one optional skin benefit agent, and the optional skin benefit agent is a water-soluble moisturizing agent, occlusive agents, plant extracts, vitamins and minerals, resorcinols, optical agents, even tone agents, anti-inflammatory agents, sunscreens and photostabilizers, antioxidants, wrinkle-reducing agents, desquamation promoters, and/or mixtures thereof.

12. The composition according to any of the preceding claims further comprising at least one of a fragrance, a fixative, an opacifier, a chelator, and/or an emollient.

13. The composition according to any of the preceding claims, wherein the composition has a pH from 4.25 to 5.25.

14. A non-therapeutic use of the composition according to any of claims 1 to 13 as an anti-acne composition or to obtain an anti-acne benefit.

15. A non-therapeutic method for reducing, treating or preventing the occurrence of acne comprising:
a. applying the composition as defined in any of claims 1 to 13 onto a surface of skin;
b. optionally, rubbing the composition into the surface of the skin; and
c. optionally repeating step (a), and optionally step (b), daily, twice a day, or three times a day.

## Patentansprüche

1. Zusammensetzung, umfassend:
a) 90 bis 93 Gew.-% Wasser;
b) 1 bis 3 Gew.-% eines nichtionischen Tensids mit einem HLB-Wert von 15,7 bis 17,5; und
c) 0,75 bis 2 Gew.-% eines Anti-Akne-Mittels, wobei das Anti-Akne-Mittel Salicylsäure ist;
wobei die Zusammensetzung im Wesentlichen frei von Verdickungsmitteln und Geliermitteln ist, vorzugsweise 0 Gew.-% Verdickungsmittel und Geliermittel enthält; und
wobei die Zusammensetzung stabiles mizellares Wasser ist, das eine kontinuierliche monophasige Wasserphase darstellt.

2. Zusammensetzung nach Anspruch 1, wobei der HLB-Wert des nichtionischen Tensids zwischen 15,7 und 16,9 liegt.

3. Zusammensetzung nach Anspruch 1 oder 2, wobei das nichtionische Tensid Ceteth-20, Laureth-23, Polysorbat-20 und/oder Mischungen davon, vorzugsweise Polysorbat-20, ist.

4. Zusammensetzung nach einem der vorhergehenden Ansprüche, wobei die Zusammensetzung 1,5% bis 2,5% des nichtionischen Tensids umfasst.

5. Zusammensetzung nach einem der vorhergehenden Ansprüche, wobei die Zusammensetzung 0,001% bis 2% oder 0,05% bis 1,0% oder 0,5% bis % oder 0,75% bis 1,5% oder 0,75% bis 1,25%, bezogen auf das Gewicht des Anti-Akne-Mittels, enthält.

6. Zusammensetzung nach einem der vorhergehenden Ansprüche, wobei die Zusammensetzung im Wesentlichen frei von Parabenen, Silikonen und/oder Sulfaten ist.

7. Zusammensetzung nach einem der vorhergehenden Ansprüche, wobei das Gewichtsverhältnis von nichtionischem Tensid zum Anti-Akne-Mittel 5:1 oder 3:1 oder 1,5:1 beträgt.

8. Zusammensetzung nach einem der vorhergehenden Ansprüche, wobei die Zusammensetzung transparent ist.

9. Zusammensetzung nach einem der vorhergehenden Ansprüche, die ferner mindestens ein optionales Tensid, vorzugsweise ein kationisches Tensid, umfasst.

10. Zusammensetzung nach einem der vorhergehenden Ansprüche, die ferner ein Polyol umfasst und das Polyol Sorbit, Glycerin, Mannit, Xylit, Maltit, Dipropylenglykol, Polypropylenglykol, Polyethylenglykol, Hydroxypropylsorbit, Hexylenglykol, 1,3-Butylenglykol, 1,2-Octandiol, 1,2-Hexandiol, Isoprenglykol, 1,2,6-Hexantriol, ethoxyliertes Glycerin, propoxyliertes Glycerin und/oder Mischungen davon ist, vorzugsweise Glycerin, Hexylenglykol und /oder Mischungen davon ist.

11. Zusammensetzung nach einem der vorhergehenden Ansprüche, ferner umfassend mindestens ein optionales Hautpflegemittel, wobei das optionale Hautpflegemittel ein wasserlöslicher Feuchtigkeitsspender, Okklusivmittel, Pflanzenextrakte, Vitamine und Mineralien, Resorcine, optische Mittel, Mittel zum Hauttonausgleich, entzündungshemmende Mittel, Sonnenschutzmittel und Photostabilisatoren, Antioxidantien, Mittel zur Faltenreduzierung, Desquamationsförderer und/oder Mischungen davon darstellt.

12. Zusammensetzung nach einem der vorhergehenden Ansprüche, ferner umfassend mindestens einen Duftstoff, ein Fixiermittel, ein Trübungsmittel, einen Chelatbildner und/oder einen Weichmacher.

13. Zusammensetzung nach einem der vorhergehenden Ansprüche, wobei die Zusammensetzung einen pH-Wert von 4,25 bis 5,25 aufweist.

14. Nicht-therapeutische Verwendung der Zusammensetzung nach einem der Ansprüche 1 bis 13 als Anti-Akne-Zusammensetzung oder zur Erzielung einer Anti-Akne-Wirkung.

15. Nicht-therapeutisches Verfahren zur Verringerung, Behandlung oder Vorbeugung des Auftretens von Akne, umfassend:
a. Auftragen der Zusammensetzung, wie in einem der Ansprüche 1 bis 13 definiert, auf die Hautoberfläche;
b. optional Einreiben der Zusammensetzung in die Hautoberfläche; und
c. optional Wiederholen von Schritt (a) und optional Schritt (b), täglich, zweimal am Tag oder dreimal am Tag.

## Revendications

1. Composition comprenant :
a) 90 % à 93 % en poids d'eau ;
b) 1 % en poids à 3 % en poids d'un tensioactif non ionique ayant un HLB de 15,7 à 17,5 ; et
c) 0,75 % à 2 % en poids d'un agent anti-acné, où l'agent anti-acné est l'acide salicylique
où la composition est sensiblement exempte d'agents épaississants et d'agents gélifiants, en ayant de préférence 0 % en poids d'agents épaississants et d'agents gélifiants ; et
où la composition est une eau micellaire stable qui est une phase aqueuse monophasique continue.

2. Composition selon la revendication 1, dans laquelle le HLB du tensioactif non ionique va de 15,7 à 16,9.

3. Composition selon la revendication 1 ou 2, dans laquelle le tensioactif non ionique est ceteth-20, laureth-23, polysorbate-20 et/ou des mélanges de ceux-ci, de préférence le polysorbate-20.

4. Composition selon l'une quelconque des revendications précédentes, où la composition comprend 1,5 % à 2,5 % du tensioactif non ionique.

5. Composition selon l'une quelconque des revendications précédentes, où la composition comprend 0,001 % à 2 %, ou 0,05 % à 1,0 %, ou 0,5 % à %, ou 0,75 % à 1,5 %, ou 0,75 % à 1,25 %, en poids de l'agent anti-acné.

6. Composition selon l'une quelconque des revendications précédentes, où la composition est sensiblement exempte de parabènes, de silicones et/ou de sulfates.

7. Composition selon l'une quelconque des revendications précédentes, où un rapport en poids du tensioactif non ionique sur l'agent anti-acné est de 5 : 1, ou 3 : 1, ou 1,5 : 1.

8. Composition selon l'une quelconque des revendications précédentes, où la composition est transparente.

9. Composition selon l'une quelconque des revendications précédentes, comprenant en outre au moins un tensioactif optionnel, de préférence un tensioactif cationique.

10. Composition selon l'une quelconque des revendications précédentes, comprenant en outre un polyol et le polyol est le sorbitol, le glycérol, le mannitol, le xylitol, le maltitol dipropylène glycol, le polypropylène glycol, le polyéthylène glycol, l'hydroxypropyl sorbitol, l'hexylène glycol, le 1,3-butylène glycol, le 1,2-octane diol, le 1,2-hexane diol, l'isoprène glycol, le 1,2,6-hexanetriol, le glycérol éthoxylé, le glycérol propoxylé et/ou des mélanges de ceux-ci, de préférence le glycérol, l'hexylène glycol et/ou des mélanges de ceux-ci.

11. Composition selon l'une quelconque des revendications précédentes, comprenant en outre au moins un agent bénéfique pour la peau optionnel, et l'agent bénéfique pour la peau optionnel est un agent hydratant soluble dans l'eau, des agents occlusifs, des extraits végétaux, des vitamines et des minéraux, des résorcinols, des agents optiques, des agents de teint unifié, des agents anti-inflammatoires, des écrans solaires et des photostabilisants, des antioxydants, des agents anti-ride, des promoteurs de desquamation et/ou des mélanges de ceux-ci.

12. Composition selon l'une quelconque des revendications précédentes, comprenant en outre au moins un élément parmi un parfum, un fixateur, un opacifiant, un chélateur et/ou un émollient.

13. Composition selon l'une quelconque des revendications précédentes, où la composition a un pH de 4,25 à 5,25.

14. Utilisation non thérapeutique de la composition selon l'une quelconque des revendications 1 à 13 en tant que composition anti-acné ou pour obtenir un bénéfice anti-acné.

15. Méthode non thérapeutique pour réduire, traiter ou prévenir l'apparition de l'acné comprenant :
a. l'application de la composition telle que définie selon l'une quelconque des revendications 1 à 13 sur une surface de la peau ;
b. éventuellement, le frottement de la composition sur la surface de la peau ; et
c. éventuellement la répétition de l'étape (a), et éventuellement de l'étape (b), une fois par jour, deux fois par jour, ou trois fois par jour.
